Europäisches Patentamt

European Patent Office

Office européen des brevets

(19)

(11) Veröffentlichungsnummer: **0 363 510 B1**

# EUROPÄISCHE PATENTSCHRIFT

(12)

(45) Veröffentlichungstag der Patentschrift: **15.12.93**

(51) Int. Cl.⁵: **G01N 33/80**, G01N 33/545

(21) Anmeldenummer: **88116911.4**

(22) Anmeldetag: **12.10.88**

(54) **Verfahren zur Suche und Identifizierung von erythrozytären Antikörpern mit Hilfe der Festphasen-Methode.**

(43) Veröffentlichungstag der Anmeldung:
**18.04.90 Patentblatt 90/16**

(45) Bekanntmachung des Hinweises auf die
Patenterteilung:
**15.12.93 Patentblatt 93/50**

(84) Benannte Vertragsstaaten:
**AT BE CH DE ES FR GB GR IT LI LU NL SE**

(56) Entgegenhaltungen:
EP-A- 0 022 669          EP-A- 0 058 780
EP-A- 0 084 102          EP-A- 0 223 978
WO-A-85/01354            WO-A-86/05591
GB-A- 2 117 514          US-A- 4 328 183

(73) Patentinhaber: **Biotest AG**
**Flughafenstrasse 4**
**D-60528 Frankfurt(DE)**

(72) Erfinder: **Uthemann, Horst, Dr. Dipl.-Chem.**
**Sachsenhäuser Landwehrweg 66**
**D-6000 Frankfurt am Main 70(DE)**

(74) Vertreter: **Beil, Hans Chr., Dr. et al**
**BEIL, WOLFF & BEIL,**
**Rechtsanwälte,**
**Postfach 80 01 40**
**D-65901 Frankfurt (DE)**

**Beschreibung**

Die Erfindung betrifft ein Verfahren zur Suche und Identifizierung von erythrozytären Antikörpern gemäß dem Oberbegriff des Anspruchs 1 sowie einen Träger und ein Kit zur Durchführung des Verfahrens.

In den letzten Jahrzehnten stellte die Agglutinationsreaktion aufgrund ihrer Einfachheit und Vielseitigkeit eine der gebräuchlichsten Methoden zur Blutgruppenbestimmung dar. Einer ihrer wesentlichen Nachteile besteht jedoch im Fehlen eines objektiven Endpunktes, der leicht automatisch oder auch visuell betimmt werden kann, da innerhalb der Verdünnungsreihen unterschiedlich starke bzw. schwache Reaktionen erfolgen, wobei z.B. schwache Reaktionen als falsch negativ angesehen werden können.

In jüngerer Zeit wurden als Alternative zu Agglutinationsreaktionen Festphasen-Methoden zur Suche und Identifizierung von erythrozytären Antikörpern sowie zur Kreuzprobe beschrieben.

Entsprechend den Publikationen von Plapp et al: A solid phase antibody screen. Am J Clin Pathol 1984; 82: 719 (1). Beck et al: Semiautomated solid phase adherence assays for pretransfusion testing. Med Lab Sci 1984; 41: 374 (2) und Capture-R solid phase assay system, Immucor 1987 (3) besteht die reaktive Festphasen-Schicht z.B. aus an den Wandungen der Näpfchen von Mikrotiterplatten aus Polystyrol immobilisierten Erythrozyten mit ausgewählten antigenen Mustern. An diese Erythrozyten können spezifisch Antikörper aus Seren oder Plasmen gebunden werden. Diese Beladung der Zellen mit Antikörpern kann mit sogenannten Indikatorzellen (IgG-beladenen Erythrozyten) sichtbar gemacht werden, indem diese Indikatorzellen über Anti-Humanglobulin (polyspezifisches oder monospezifisches Anti-Human-IgG) mit den beladenen immobilisierten Erythrozyten verknüpft werden. Es kommt zur Ausbildung einer Erythrozyten-Doppelschicht an den Wandungen der Näpfchen, wenn in den zu untersuchenden Proben ausreichende Mengen erythrozytärer Antikörper vorliegen. Negative Ergebnisse sind an Knöpfchen nicht gebundener Indikatorzellen auf dem Näpfchenboden zu erkennen.

In (2) und in Rachel et al: A solid phase antiglobulin test, Transfusion 1985, 25:24 (4) wird eine Festphasen-Methode für Kreuzproben beschrieben, die Mikrotiterplatten verwendet, deren Näpfchen mit humanem IgG beschichtet sind. Nach einer Vorinkubation von Erythrozyten mit Seren oder Plasmen, die Antikörper gegen Erythrozyten enthalten können, werden die beladenen bzw. unbeladenen Erythrozyten gewaschen und mit einem Anti-Human-Reagenz in die mit IgG beschichtete Mikrotiterplatte übertragen. Über die Anti-Human-IgG-Komponente des Reagenzes werden mit Antikörpern vom IgG-Typ beladene Erythrozyten als sichtbarer Film an die feste Phase gebunden. Auch hier bilden unbeladene Erythrozyten Knöpfchen sedimentierter Zellen.

Die EP-A-0058780 beschreibt den Nachweis von Antigenen oder Antikörpern in Behältern, die mit Immunglobulin-bindenden Komponenten (z.B. Protein A) beschichtet sind. Zum Nachweis der Rhesusblutgruppen werden dazu z.B. Spenderblute mit den entsprechenden Antiseren vorinkubiert.

Diesen Methoden ist gemeinsam, daß sich positive Reaktionsmuster als Schicht spezifisch gebundener Erythrozyten an speziell präparierten festen Phasen darstellen, während negative Reaktionen nicht zur Ausbildung einer Festphasenschicht führen. Die unterschiedlichen Reaktionsmuster lassen sich visuell oder spektrophotometrisch ablesen, und man kommt zu einer objektiven Ja- oder Nein-Bestimmung.

Der Nachteil der bisher beschriebenen Festphasen-Methoden (IgG-beladene Indikatorzellen, IgG-beschichtete Näpfchen) besteht jedoch darin, daß nur erythrozytäre Antikörper vom IgG-Typ nachgewiesen werden können, wodurch das Anwendungsspektrum dieser Methoden begrenzt ist.

Der Erfindung liegt die Aufgabe zugrunde, ein Verfahren zur Suche und Identifizierung von erythrozytären Antikörpern im Rahmen der Festphasen-Methode, sowie Hilfsmittel zur Durchführung des Verfahrens bereitzustellen, mit dessen Hilfe nicht nur erythrozytäre Antikörper vom IgG-Typ gefunden werden bzw. Erythrozyten erkannt werden, die mit IgG beladen sind, sondern auch Antikörper vom Typ IgM oder IgA gefunden werden bzw. Erythrozyten erkannt werden, die mit Komplement beladen sind.

Diese Aufgabe wird bei einem gattungsgemäßen Verfahren durch die kennzeichnenden Merkmale des Anspruchs 1 gelöst.

Nach zahlreichen vergeblichen Versuchen, eine naheliegendere Lösung der gestellten Aufgabe zu finden, war es nicht nur neu und überraschend, daß mit Hilfe einer Protein A- oder Protein G-Schicht auf einem Träger sich praktisch alle Komponenten des verwendeten polyspezifischen Anti-Human-Globulins binden ließen, sondern die Verwendung von polyspezifischem Anti-Human-Globulin für die Festphasen-Methode wurde erst attraktiv.

Wie durch nachstehende Beispiele erläutert wird, werden mit Hilfe des erfindungsgemäßen Verfahrens eine Reihe von Antikörpern erfaßt, die mit den vorstehend aufgezeigten bekannten Methoden nicht erfaßt werden.

So werden beispielsweise von den bekannten Testsystemen folgende Antikörper nicht erfaßt:

Literaturstelle (1):

z.B. Anti-Le$^a$, Anti-Le$^b$, Anti-Le$^{a+b}$ und Anti-Kn$^a$ vom IgM-Typ, (Tabelle 2);

(2): z.B. Anti-Le$^a$, Anti-Le$^b$, Anti-Le$^{a+b}$ und Anti-Kn$^a$ vom IgM-Typ, (Tabelle 4);

(3): z.B. Anti-Kell vom IgM-Typ, (Tabelle 2);

(4): z.B. Anti-Le$^a$ und Anti-Le$^b$ vom IgM-Typ, (Tabelle 1);

(5) Symposium: Rolih et al: Solid Phase Adherence Assays; Alternatives to Conventional Blood Bank Tests. Lab Med. Vol.16 (1985) 766 (5): z.B. Anti-Le$^a$ und Anti-Le$^b$ vom IgM-Typ, (Tabelle III).

In den nachstehenden Versuchen wurden als Träger Mikrotiterplatten aus Polystyrol verwendet, obgleich jede andere Art von Träger bzw. Trägermaterial wie sie bei Festphasen-Methoden bekannt sind, verwendet werden können. Die Protein A- oder Protein G-Schichten auf dem Träger lassen sich beispielsweise dadurch herstellen, daß man das Protein in isotoner Kochsalzlösung oder in einer geeigneten Pufferlösung löst und mit der Oberfläche eines geeigneten Trägers in Berührung bringt. Die Konzentration des Proteins in der Beschichtungslösung kann zwischen o,1 und 20 $\mu$g/ml liegen. Bei Verwendung von Mikrotiterplatten als Träger erwiesen sich etwa 10 bis 100 $\mu$l der Beschichtungslösung pro Näpfchen als zweckmäßig. Nach etwa 14 - 20-stündigem Stehen sollte die Beschichtungslösung abgesaugt und die Näpfchen 2 - 5 mal mit destilliertem Wasser, dem ggf. ein Konservierungsmittel zugesetzt wird, gewaschen werden. Die so erhaltenen beschichteten Träger können sofort verwendet werden oder nach Trocknung und geeigneter Verpackung gelagert werden.

Diesen gebrauchsfertigen Trägern, z.B. Mikrotiterplatten, brauchen zum Zwecke eines Tests nur noch die in polyspezifischem Anti-Human-Globulin resuspendierten Erythrozyten, die ggf. mit Antikörper beladen sind, zugesetzt werden.

Nachstehende Beispiele dienen der weiteren Erläuterung der Erfindung.

Beispiel 1

Beschichtung eines Trägers mit einem Protein

Ein von Zellwänden gereinigtes, lyophilisiertes, im wesentlichen salzfreies, pulverförmiges Protein A wurde in isotoner Kochsalzlösung gelöst. Die Konzentration der Beschichtungslösung betrug 0,5 $\mu$g/ml. Je 100 $\mu$l dieser Beschichtungslösung wurden in jedes Näpfchen einer Mikrotiterplatte eingefüllt. Nach 16-stündigem Stehen in einem geerdeten Beschichtungsschrank wurde die Beschichtungslösung abgesaugt, und die Näpfchen 3 mal mit destilliertem Wasser, dem ein Konservierungsmittel zugesetzt wurde, gewaschen. Nach Trocknung wurden die beschichteten Mikrotiterplatten in Aluminiumtüten eingeschweißt.

Beispiel 2

Testverfahren

Es wurden 1,0%ige Suspensionen von Such-, Identifizierungs- oder Patienten/Spender (Autokontrolle) - Erythrozyten in LISS hergestellt. Danach wurden 100$\mu$l Patienten/Spender-Seren oder -Plasmen und 100$\mu$l der Erythrozyten-Suspension in jedes Näpfchen einer Mikrotiterplatte gegeben. Nach Schütteln wurden die zugedeckten Mikrotiterplatten 30 Minuten bei 37°C in einem Brutschrank inkubiert. Anschließend wurden die Platten bei 600 x g 2 Minuten lang zentrifugiert.

Die Zellen wurden 5 bis 7 mal mit Coombs-Waschlösung gewaschen, um nicht gebundene Antikörper zu entfernen, und die Salzlösung wurde abdekantiert. Die Erythrozyten wurden durch Zugabe von 100$\mu$l eines polyspezifischen Anti-Human-Globulins folgender Zusammensetzung:

| | | |
|---|---|---|
| Anti-IgG | Titer | 1:256 |
| Anti-IgM | " | 1: 16 |
| Anti-IgA | " | 1: 16 |
| Anti-C$_3$b | " | 1: 32 |
| Anti-C$_3$d | " | 1: 32 |
| Tween 20 | 0,1 % | |

unter Verwendung einer Mehrkanalpipette resuspendiert. 100µl der resuspendierten Zellen wurden in jedes Näpfchen der gemäß Beispiel 1 beschichteten Mikrotiterplatten unter Verwendung einer Mehrkanalpipette gegeben. Vor dem Ablesen ließ man die so behandelte Mikrotiterplatte 45 Minuten bei Raumtemperatur stehen.

Beispiel 3

Empfindlichkeit des erfindungsgemäßen Testverfahrens

Um die Empfindlichkeiten des erfindungsgemäßen Testverfahrens und üblicher Antiglobulintests zu vergleichen, wurden die Endpunkte von serienverdünnten Antikörpern bekannter Spezifitäten in AB-Serum parallel bestimmt. Die Endpunkte der Tests wurden interpretiert als die höchsten Verdünnungen, die klare sichtbare Reaktionen zeigten. Beim Testen eines Panels von 3 Suchzellen und 8 Identifizierungszellen bekannter antigener Zusammensetzung, wurden nur solche Antikörper als identifiziert angesehen, die mit allen antigen-positiven Zellen reagierten. Wie aus Tabelle 1 ersichtlich ist, war das erfindungsgemäße Testverfahren meistens 2 - 4 mal empfindlicher, und die Bewertung der Stärke der Reaktionen ergab wesentlich höhere Werte.

| Antikörper Spezifizität | | Konventionelle Methode | | Erfindungsgemäße Festphasenmethode | |
|---|---|---|---|---|---|
| | | Titer | Bewertung | Titer | Bewertung |
| Rh-System | | | | | |
| | D | 1024 | 42 | 8192 | 151 |
| | C | 64 | 14 | 1024 | 77 |
| | $c_w$ | 512 | 36 | 2048 | 96 |
| | $C^w$ | 64 | 27 | 1024 | 66 |
| | C + D | 8192 | 83 | 128000 | 242 |
| | E | 256 | 8 | 2048 | 55 |
| | e | 2 | 22 | 16 | 125 |
| Kell-System | | | | | |
| | K | 2048 | 26 | 4096 | 50 |
| | $k_b$ | 32 | 39 | 64 | 120 |
| | $Kp^b$ | 256 | 72 | 1024 | 201 |
| Kidd-System | | | | | |
| | $Jk^a_b$ | 8 | 49 | 32 | 82 |
| | $Jk^b$ | 8 | 13 | 16 | 44 |
| Duffy-System | | | | | |
| | $Fy^a_b$ | 16 | 67 | 128 | 142 |
| | $Fy^b$ | 4 | 19 | 16 | 94 |
| MNSs-System | | | | | |
| | M | 256 | 168 | 256 | 216 |
| | N | 16 | 111 | 16 | 120 |
| | S | 1 | 13 | 16 | 100 |
| | s | 8 | 37 | 16 | 88 |
| Lewis-System | | | | | |
| | $Le^a_b$ | 256 | 64 | 1024 | 132 |
| | $Le^b$ | 2 | 26 | 2 | 42 |
| Lutheran-System | | | | | |
| | $Lu^a_b$ | 16 | 18 | 128 | 107 |
| | $Lu^b$ | 16 | 50 | 16 | 89 |
| P-System | | | | | |
| | $P_1$ | 4 | 29 | 8 | 64 |

Tabelle 1: Vergleich der Empfindlichkeiten

Beispiel 4

Routinemäßige Suche und Identifizierung von Spender-Antikörpern

Antikörper Such- und Identifizierungs-Tests wurden parallel bei 160 Blutspenderseren und 1369 Spenderplasmen durchgeführt. Mehrfachantikörper wurden identifiziert unter Verwendung von verschiedenen Panels von Identifizierungs-Zellen. Die Ergebnisse sind in Tabelle 2 zusammengefaßt.

| | Getestete Anzahl | Positiv im Röhrchentest | % | Positiv im erfindungsgemäßen Test | % | Identifizierung |
|---|---|---|---|---|---|---|
| Blutspender | 160 | 0 | 0 | 0 | 0 | |
| Plasmaspender | 1369 | 8 | 0,58 | 8 | 0,58 | 3 anti-D<br>1 anti-CD<br>1 anti-CD, anti-Yt$^{a}$<br>1 anti-C$^{w}$<br>1 anti-e, anti-K, anti-Fy$^{a}$<br>1 anti-Le$^{a}$ |

Tabelle 2: Ergebnisse von routinemäßiger Spender-Antikörper-Suche und Identifizierung.

Beispiel 5

Untersuchung von Patientenseren

Über einen Zeitraum von 3 Monaten wurde das erfindungsgemäße Verfahren auf seine Fähigkeit getestet, Antikörper in Patientenseren zu entdecken, indem man es mit einem üblichen Röhrchentest für Antikörper-Suche und -Identifizierung verglich. Insgesamt 848 Seren, einschließlich 68 Nabelschnurseren wurden getestet mit Autokontrollen (n = 491) bzw. ohne Autokontrollen (n = 357). Die Analyse sämtlicher Ergebnisse findet sich in den Tabellen 3 und 4.

| Röhrchen | Erfin- dungsge- mäß | N | % | Identifizierung/Interpretation |
|---|---|---|---|---|
| Neg. | Neg. | 811 | 95.64 | |
| Pos. | Pos. | 14 | 1,65 | 1 Anti-D<br>2 Anti-CD<br>1 Anti-Kell<br>1 Anti-Le$^a$, nur reaktiv in NaCl,nicht reaktiv im indirekten Coombs-Tes(ICT)<br>2 Anti-Le$^a$, nur reaktiv im ICT<br>3 Kälte-Antikörper, nicht reaktiv im ICT<br>4 inkomplette Wärme-Autoantikörper bei Patienten mit Autoimmun-haemolytischer Anaemie (AIHA) |
| Neg. | Pos. | 20 | 2,36 | 1 Anti-C<br>1 Anti-Kell<br>1 inkompletter Wärme-Antikörper bei einem Patienten mit AIHA<br>1 Kälte-Antikörper<br>16 Antikörper, ohne genaue Identifizierung |
| Pos. | Neg. | 3 | 0,35 | 3 falsche positive Ergebnisse, negativ bei wiederholtem Testen |

Tabelle 3: Antikörper in Patientenseren.

EP 0 363 510 B1

| Röhrchen | erfindungs-gemäß | N | % | Identifizierung/Interpretation |
|---|---|---|---|---|
| Pos. | Pos. | 7 | 1.43 | Direkt AHG positiv: 5 (AIHA)  Direkt AHG negativ: 2 |
| Neg. | Pos. | 11 | 2.24 | Direkt AHG positiv: 2 (1 nur $C_3$d positiv, 1 IgD positiv, AIHA)  Direkt AHG negativ: 9 (weitere Studien erforderlich) |
| Pos. | Neg. | 3 | 0.61 | Direkt AHG negativ: 3 falsch positiv |
| Total | | 21 | 4.28 | |

Tabelle 4: Autoantikörper in Patientenseren.

## Patentansprüche

1. Verfahren zur Suche und Identifizierung von erythrozytären Antikörpern mit Hilfe der Festphasen-Methode, bei dem man Erythrozyten mit ausgewählten antigenen Mustern mit den auf Antikörper zu

9

untersuchenden Seren oder Plasmen in Kontakt bringt und mit Hilfe eines Anti-Human-Globulin-Reagenz auf einen beschichteten Träger überträgt, dadurch gekennzeichnet, daß man polyspezifisches Anti-Human-Globulin der Zusammensetzung Anti-IgG, Anti-IgM, Anti-IgA, Anti-$C_3$b und Anti-$C_3$d zum Übertragen der Such- bzw. Identifizierungszellen oder der Eigenzellen bei Autokontrollen verwendet und daß man einen Träger verwendet, dessen Beschichtung aus Protein A oder Protein G besteht.

2.  Verfahren nach Anspruch 1, dadurch gekennzeichnet, daß man als Träger Mikrotiterplatten verwendet.

3.  Verfahren nach einem der vorstehenden Ansprüche, dadurch gekennzeichnet, daß man als Trägermaterial Polystyrol verwendet.

4.  Träger zur Durchführung des Verfahrens nach Anspruch 1, an dessen Oberfläche sich eine Schicht aus Protein A oder Protein G befindet.

5.  Kit zur Durchführung des Verfahrens nach Anspruch 1, enthaltend einen beschichteten Träger gemäß Anspruch 4 und ein polyspezifisches Anti-Human-Globulin-Reagenz der Zusammensetzung Anti-IgG, Anti-IgM, Anti-IgA, Anti-$C_3$b und Anti-$C_3$d.

## Claims

1.  A process for the search and identification of erythrocytic antibodies by the solid phase method in which erythrocytes with selected antigenic patterns are brought into contact with the sera or plasmas to be investigated for antibodies and are transferred to a coated carrier by means of an Anti-Human Globulin reagent, characterised in that polyspecific Anti-Human-Globulin having the composition Anti-IgG, Anti-IgM, Anti-IgA, Anti-$C_3$b and Anti-$C_3$d is used for transferring the search and identification cells or the own cells in the case of auto controls and in that the carrier used is one whose coating consists of protein A or protein G.

2.  A process according to Claim 1, characterised in that microtitre plates are used as carriers.

3.  A process according to one of the preceding claims, characterised in that the carrier material used is polystyrene.

4.  A carrier for carrying out the process according to Claim 1, on the surface of which is situated a layer of protein A or protein G.

5.  A kit for carrying out the process according to Claim 1, containing a coated carrier according to Claim 4 and a polyspecific Anti-Human-Globulin reagent having the composition Anti-IgG, Anti-IgM, Anti-IgA, Anti-$C_3$b and Anti-$C_3$d.

## Revendications

1.  Procédé de recherche et d'identification d'anticorps érythrocytaires à l'aide de la méthode en phase solide dans laquelle on met en contact des érythrocytes ayant les types antigéniques choisis avec des sérums et des plasmas dans lesquels on recherche les anticorps, et on les transfère à l'aide d'un réactif anti-globulines humaines sur un support revêtu, caractérisé en ce que l'on utilise un réactif anti-globulines humaines polyspécifique composé d'anti-IgG, d'anti-IgM, d'anti-IgA, d'anti-$C_3$b et d'anti-$C_3$d pour transférer les cellules de recherche ou d'identification ou les cellules propres lors d'autocontrôles, et en ce que l'on utilise un support dont le revêtement est constitué de protéine A ou de protéine G.

2.  Procédé selon la revendication 1, caractérisé en ce que l'on utilise des plaques de microtitrage comme supports.

3.  Procédé selon l'une des revendications précédentes, caractérisé en ce qu'on utilise du polystyrène comme matière du support.

4.  Support pour la mise en oeuvre du procédé selon la revendication 1, sur la surface duquel se trouve une couche de protéine A ou de protéine G.

5. Trousse pour la mise en oeuvre du procédé selon la revendication 1, contenant un support revêtu selon la revendication 4 et un réactif anti-globulines humaines polyspécifique composé d'anti-IgG, d'anti-IgM, d'anti-IgA, d'anti-$C_3$b et d'anti-$C_3$d.